# EUROPEAN PATENT SPECIFICATION

(11) **EP 1 471 908 B1**
(45) Date of publication and mention of the grant of the patent: **08.08.2007**
(21) Application number: 03776442.0
(22) Date of filing: 17.10.2003
(51) Int. Cl.: A61K 31/4178, A61K 9/14

(54) **PROCESS FOR PREPARING LOSARTAN POTASSIUM WITH IMPROVED FLOWABILITY**
VERFAHREN ZUR HERSTELLUNG VON LOSARTAN KALIUMSALZ MIT VERBESSERTEN FLIESSEIGENSCHAFTEN
PROCEDE POUR PREPARER DU POTASSIUM DE LOSARTAN AVEC COULABILITE AMELIOREE

(30) Priority: 17.10.2002 US 419450 P; 12.11.2002 US 426072 P; 14.11.2002 US 426461 P; 04.12.2002 US 431450 P; 09.12.2002 US 431809 P
(43) Date of publication of application: 03.11.2004
(73) Proprietor: TEVA PHARMACEUTICAL INDUSTRIES LTD., 49131 Petah Tiqva (IL); Teva Pharmaceuticals USA, Inc., North Wales, PA 19454-1090 (US)
(72) Inventor: LIFSHITZ, Igor, Petach-Tiqva (IL); KOR, Ilan, IL- Shoham (IL); SHABAT, Shalom, IL- Yavne (IL)
(74) Representative: Bradley, Josephine Mary
(86) International application number: PCT/US2003/032885
(87) International publication number: WO 2004/035049

(56) References cited:
- WO-A-03/048135
- US-A- 5 859 258

## Description

### RELATED APPLICATIONS

The present Application claims the benefit of the filing date of the following United States Provisional Patent Applications No. 60/419,450, filed October 17, 2002; No. 60/426,072, filed November 12, 2002; No. 60/426,461, filed November 14, 2002; No. 60/431,450, filed December 4, 2002 and No. 60/431,809, filed December 9,2002.

### FIELD OF THE INVENTION

The present invention relates to a new process for preparing, and to compositions containing, losartan potassium with improved flowability.

### BACKGROUND OF THE INVENTION

Losartan potassium, also known as 2-butyl-4-chloro- 1 -[[2'-(1H-tetrazol-5-yl)[1, 1'-buphenyl]-4-yl]-1 H-imidazole-5-methanol monopotassium salt, is a competitive AT₁, angiotensin II receptor antagonist. Activation of AT₁, receptors in the outer membrane of vascular smooth muscle cells of the heart and arteries causes the tissues to constrict. AT₁, receptors are activated by an octa-peptide, angiotensin II. Angiotensin II helps to maintain constant blood pressure despite fluctuations in a person's state of hydration, sodium intake and other physiological variables. Angiotensin II also performs the regulatory tasks of inhibiting excretion of sodium by the kidneys, inhibiting norephedrin reuptake and stimulating aldosterone biosynthesis. By inhibiting angiotensin II binding to AT₁ receptors, losartan disrupts the vasoconstriction mediated by AT₁ receptors. Blocking vasoconstriction by angiotensin II has been found to be beneficial to patients with hypertension.

In 1995, losartan became the first nonpeptide AT₁ antagonist approved by the U.S. Food and Drug Administration for clinical use. In particular, losartan is approved for the treatment of hypertension alone or in combination with other antihypertensive agents. Losartan may be administered orally as its monopotassium salt. Losartan potassium is available by prescription in tablet form as a sole active ingredient (Cozaar^{®}: Merck) and as a co-active ingredient with hydrochlorothiazide (Hyzaar^{®}: Merck).

The present invention relates to the solid state physical properties of losartan potassium. These properties can be influenced by controlling the conditions under which losartan potassium is obtained in solid form. Solid state physical properties include, for example, the flowability of the milled solid. Flowability affects the ease with which the material is handled during processing into a pharmaceutical product. When particles of the powdered compound do not flow past each other easily, a formulation specialist must take that fact into account in developing a tablet or capsule formulation, which may necessitate the use of glidants such as colloidal silicon dioxide, talc, starch or tribasic calcium phosphate.

Losartan potassium can be prepared by a variety of methods. For instance, in U.S. Patent Nos. 5,128,355, 5,138,069 and 5,155,118, Example 316, Parts C and D respectively in all, trityl losartan (1-[(2'-(triphenylmethyltetrazol-5-yl)-biphenyl-4-yl)-methyl]-2-butyl-4-chloro-5-hydroxymethylimidazole) was deprotected with a mixture of hydrochloric acid and methanol to form losartan free acid (2-butyl-4-chloro-1-[[2'-(1H-tetrazol-5-yl)[1,1'-buphenyl]-4-yl]-1 H-imidazole-5-methanol). Losartan potassium was formed by reacting losartan free acid with potassium hydroxide in a mixture of isopropyl alcohol and heptane.

In U.S. Patent No. 5,962,500, Example 5, and U.S. Patent Nos. 5,206,374 and 5,310,928, Example 21 in both, trityl losartan was deprotected with a mixture of aqueous sulfuric acid and tetrahydrofuran (THF), from which the salt was generated by extracting losartan from the mixture with an adsorbent, treating the adsorbent with dipotassium hydrogen phosphate and eluting losartan potassium from the adsorbent with 20% aqueous THF. The eluent was then concentrated and diluted with isopropyl alcohol, which yielded crystalline losartan potassium. Alternatively, the product was isolated by spray drying.

In U.S. Patents Nos. 5,130,439, 5,206,374 and 5,310,928, Example 8 in all, trityl losartan was deprotected with a mixture of aqueous hydrochloric acid and THF to form losartan free acid. Losartan potassium was formed by reacting losartan free acid with potassium hydroxide in a mixture of isopropyl alcohol, water and heptane.

Crystalline losartan potassium made from the processes described above is hygroscopic and has poor powder flow characteristics. Because of this poor flowability, problems occur with handling and processing during milling and formulating. Solids that are fine, loose powders often have poor flow characteristics and are resistant to blending and dispersion in liquids because the clump and wet poorly. Dust associated with fine powders can develop a static charge and cling to equipment, making handling and feeding through volumetric equipment difficult.

In the past, powders with poor flow properties have been granulated to vary their particle size distribution in order to improve their characteristics. Other methods used to improve the flow properties of powders have been to treat the surface of the powdered material during manufacturing or to apply a lubricant to a powdered material that was to be subsequently processed.

The flowability of losartan potassium can be measured using the Hausner ratio, wherein a known weight of material is poured into a measuring cylinder, the volume recorded, and the poured density calculated. The cylinder is then tapped against a surface for a specified number of times, the new volume again recorded, and the tapped density calculated. The Hausner ratio is equal to tapped density divided by poured density. Henry H. Hausner, "Fiction Conditions in a Mass of Metal Powders," Int. J. Powder Metall. vol. 3, 1967, pp. 7-13. A ratio of <1.3 indicates a free flowing material while a ratio of >1.5 indicates a poor flowing material.

The flowability of dry crystals depends on many parameters such as crystal density, crystal size distribution, median crystal size, shape of the crystals, voidage fraction of the solids, degree of mixedness, inner voidage of crystals, residual moisture content, and concentration of adsorbed vapors and gases. A. Weissberger, II Organic Solvents, Physical Properties and Methods of Purification, 315 (4th Ed. 1986). The smaller the particles and the more the particles deviate from spheres, the stronger the friction and cohesion forces are, which results in reduced flowability. Further, the flowability of solid material may depend with time because parameters such as voidage fraction, interparticle forces and crystalline bridges, and adsorbates change with time and will influence such flowability.

To obtain more preferred crystals of losartan potassium, U.S. Patent No. 5,859,258 discloses the use of an antisolvent, to control the approach to saturation and to control crystal growth, combined with massive seeding.

### SUMMARY OF THE INVENTION

In one aspect, the present invention relates to a method of increasing the flowability of losartan potasium powder initially having a Hausner ratio > 1.45 including the step of reslurrying such losartan potassium powder, especially such losartan potassium powder made by neutralization of the free acid in the presence of isopropanol, in a reslurry solvent selected from the hydrocarbons (especially the heptanes, cyclohexane, or toluene), the alkyl ethers, the alkyl esters, and mixtures of two or more of these. The method further includes the steps of isolating, drying, and, optionally, milling the losartan potassium. Losartan potassium so treated has Hausner ratio < 1.45, especially ≤1.3.
In another aspect, the present invention relates to a method of increasing the flowability of losartan potassium initially having a Hausner ratio > 1.45 including the steps of reslurrying such losartan potassium powder, especially such losartan potassium powder made by neutralization of the free acid in the presence of isopropanol, in a reslurry solvent selected from the heptanes, cyclohexane, and toluene. The method further includes the steps of isolating, drying, and milling the reslurried losartan potasium so treated. Losartan potasium so treated has Hausner ratio < 1.45, especially ≤ 1.3.

### DETAILED DESCRIPTION OF THE INVENTION

Losartan potassium that has been isolated (e.g. crystallized) by procedures using protic solvents in work-up or isolation has poor flowability, as indicated by a Hausner ratio of about 1.45-1.90. Powder of crystals of losartan potassium that has been subjected to the treatment method of the present invention has improved flowability as indicated by a Hausner ratio of about 1.3-1.45. A low Hausner ratio corresponds with crystals having high flowability. Measurement of Hausner ratio is well-known in the art and is described, for example, by Mersmann; Crystallization Technology Handbook (A. Mersmann, ed., 2nd ed., Marcel Dekker)

In one embodiment, the present invention provides a method for improving the flowability of crystals of losartan potassium having an initial Hausner ratio ≥ 1.45 that includes the step of reslurring crystals of losartan potassium in a reslurry solvent, preferably a hydrocarbon, alkyl ether, or alkyl ester reslurry solvent, whereby a slurry is obtained. In particular, the reslurring step involves contacting, with agitation, solid losartan potassium with a reslurry solvent in which losartan potassium is at most partially soluble.

The reslurrying can be carried-out in any convenient equipment. The reslurrying is preferably carried-out at the boiling point of the reslurry solvent. In this case, the refluxing action of the reslurry slovent can provide the agitation, but mechanical agitation can also be used.

The reslurry solvent is used in an amount of at least about 2 volumes or more, preferably more. "Volumes" is defined as 1 mL solvent per 1 gram of losartan potassium, *e.g.* "10 volumes" means 10 mL solvent per 1 gram of losartan potassium.

Reslurry solvents useful in the practice of the present invention include hydrocarbons, both aliphatic and aromatic. Examples of aliphatic hydrocarbons (i.e. alkanes) include the hexanes and the heptanes. Toluene is an example of an aromatic hydrocarbon. The aliphatic hydrocarbons can be essentially a single structural isomer (e.g. *n*-heptane), or they can comprise a mixture of structural isomers. The skilled artisan recognizes that the aliphatic hydrocarbons of commerce can and frequently do comprise a mixture of normal and branched structures and such mixtures are useful in the practuce of the present invention. Accordingly, as used herein, phrases such as "the heptanes" refers to a single isomer (e.g. n-heptane) as well as to mixtures of structural isomers (e.g. 2-methylhexane).

Cyclic alkanes such as cyclohexane and methylcyclohexane are also useful reslurry solvents that are hydrocarbons. Benzene, toluene, and the xylenes are aromatic hydrocarbons useful as reslurry solvents in the practice of the present invention.

The aliphatic ethers (dialkyl ethers) are also useful as reslurry solvents in the practice of the present invention. Aliphatic ethers have the structural formula CₙH₂ₙ₊₁-O-CₘH₂ₘ₊₁, wherein n and m are independently 2 to 6. Diethyl ether and methyl t-butyl ether are examples of aliphatic ethers useful in the practice of the present invention.

The alkyl esters of aliphatic carboxylic acids, especially acetic and propanoic acids, are also useful as reslurry solvents in the practice of the present invention. The alkyl esters have the structural formula R₁-OC(O)R₂, wherein R₁ and R₂ are, independently, normal or branched C1 to C5 alkyl. Ethyl acetate (R₁ = C₂H₅; R₂ = CH₃)is an example of an alkyl ester that is useful as a reslurry solvent in the practice of the present invention.

Preferred reslurry solvents include the hexanes, the heptanes, especially *n*-heptane, cyclohexane, toluene, diethyl ether, methyl t-butyl ether, ethyl acetate, and butyl acetate.

The reslurrying is carried-out for a time sufficient to effect the objectives of the present invention that can be determined by routine optimization. Reslurry times of about 3 to 10 hours are typically effective. At the end of the reslurry time, losartan potassium crystals are isolated from the slurry by known methods, for example filtration. The powder of isolated losartan potassium crystals is dried, preferably under vacuum, to obtain losartan potassium powder having improved flowability (Hausner ratio < 1.45).

The reslurry produces best results when the temperature of the reslurry is higher and the duration of the reslurry is longer. The resulting losartan potassium can be isolated and dried by methods known in the art.

Examples of starting materials for the novel process include losartan potassium obtained by any of the methods described in the patents previously discussed, which employ protic solvents, *i.e.* U.S. Patents Nos. 5,128,355; 5,130,439; 5,138,069; 5,155,118; 5,206,374; 5,310,928; 5,608,075; 5,663,187; 5,663,186 and 5,962,500

The method of the present invention operates on losartan potasium of Hausner ratio ≥ 1.45 from any source. However, the benefits of the present invention are most prominent when losartan potassium made by a method that employs protic solvents is the starting material. For example, a preferred starting material is losartan potassium made from losartan free acid, wherein losartan free acid is contacted with a potassium base (i.e. a base having a potassium cation) in the presence of isopropyl alcohol. Potassium hydroxide is a preferred potassium base. Losartan potassium can be made via losartan free acid, preferably by contacting losartan free acid with a mixture of isopropyl alcohol and an antisolvent as discussed U.S. Patent No. 5,310,928 ("the '928 patent)", which is hereby incorporated by reference. Another preferred losartan free acid is made from trityl losartan, wherein trityl losartan is contacted with a mixture of water, acetone and sulfuric acid. Losartan free acid is also preferably made from trityl losartan, wherein trityl losartan is contacted with a mixture of water, THF, and sulfuric acid as discussed in the '928 patent.

Dried losartan potassium treated by the reslurry method of the present invention can be milled, for example using a cone mill, in order to delump the material and effect moderate size reduction. After such milling, the losartan potassium exhibits improved flowability properties.

The losartan potassium treated by the method of the present invention may be incorporated into pharmaceutical compositions. Such pharmaceutical compositions contain losartan potassium having improved flowability, optionally in mixture with other active ingredients such as hydrochlorothiazide. In addition to the active ingredient(s), the pharmaceutical compositions of the present invention can contain one or more excipients, such as diluents, binders, disintegrants, glidants, and lubricants.

Diluents increase the bulk of a solid pharmaceutical composition and can make a pharmaceutical dosage form containing the composition easier for the patient and caregiver to handle. Diluents for solid compositions include, for example, microcrystalline cellulose, microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates, potassium chloride, powdered cellulose, sodium chloride, sorbitol and talc.

Solid pharmaceutical compositions that are compacted into a dosage form like a tablet can include excipients whose functions include helping to bind the active ingredient and other excipients together after compression. Binders for solid pharmaceutical compositions include acacia, alginic acid, carbomer, carboxymethylcellulose sodium, dextrin, ethyl cellulose, gelatin, guar gum, hydrogenated vegetable oil, hydroxyethyl cellulose, hydroxypropyl cellulose, hydroxypropyl methyl cellulose, liquid glucose, magnesium aluminum silicate, maltodextrin, methylcellulose, polymethacrylates, povidone, pregelatinized starch, sodium alginate and starch.

The dissolution rate of a compacted solid pharmaceutical composition in the patient's stomach can be increased by the addition of a disintegrant to the composition. Disintegrants include alginic acid, carboxymethylcellulose calcium, carboxymethylcellulose sodium, colloidal silicon dioxide, croscarmellose sodium, crospovidone, guar gum, magnesium aluminum silicate, methyl cellulose, microcrystalline cellulose, polacrilin potassium, powdered cellulose, pregelatinized starch, sodium alginate, sodium starch glycolate and starch.

Glidants can be added to improve the flow properties of non-compacted solid composition and improve the accuracy of dosing. Excipients that can function as glidants include colloidal silicon dioxide, magnesium trisilicate, powdered cellulose, starch, talc and tribasic calcium phosphate.

When a dosage form such as a tablet is made by compaction of a powdered composition, the composition is subjected to pressure from a punch and dye. Some excipients and active ingredients have a tendency to adhere to the surfaces of the punch and dye, which can cause the product to have pitting and other surface irregularities. A lubricant can be added to the composition to reduce adhesion and ease release of the product form the dye. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc and zinc stearate.

Selection of excipients and the amounts to use can be readily determined by the formulation scientist based upon experience and standard procedures in the field.

The solid compositions of the present invention include powders, granulates, aggregates and compacted compositions.

Losartan potassium treated by the method of the present invention can be administered for treatment of hypertension by any means that delivers the active pharmaceutical ingredient(s) to the site of the body where competitive inhibition of an AT₁, receptor exerts a therapeutic effect on the patient. For example, administration can be oral, buccal, parenteral (including subcutaneous, intramuscular, and intravenous) rectal, inhalant and ophthalmic. Although the most suitable route in any given case will depend on the nature and severity of the condition being treated, the most preferred route of the present invention is oral. Losartan potassium treated by the method of the present invention can be conveniently administered to a patient in oral unit dosage form and prepared by any of the methods well-known in the pharmaceutical arts. Dosage forms include solid dosage forms like tablets, powders, capsules, sachets, troches and lozenges.

The active ingredient(s) and excipients can be formulated into compositions and dosage forms according to methods known in the art.

A composition for tableting or capsule filing can be prepared by wet granulation. In wet granulation some or all of the active ingredients and excipients in powder form are blended and then further mixed in the presence of a liquid, typically water, that causes the powders to clump up into granules. The granulate is screened and/or milled, dried and then screened and/or milled to the desired particle size. The granulate can then be tableted or other excipients can be added prior to tableting such as a glidant and/or lubricant.

A tableting composition can be prepared conventionally by dry blending. For instance, the blended composition of the actives and excipients can be compacted into a slug or a sheet and then comminuted into compacted granules. The compacted granules can be compressed subsequently into a tablet.

As an alternative to dry granulation, a blended composition can be compressed directly into a compacted dosage form using direct compression techniques. Direct compression produces a more uniform tablet without granules. Excipients that are particularly well suited to direct compression tableting include microcrystalline cellulose, spray dried lactose, dicalcium phosphate dihydrate and colloidal silica. The proper use of these and other excipients in direct compression tableting is known to those in the art with experience and skill in particular formulation challenges of direct compression tableting.

A capsule filling can comprise any of the aforementioned blends and granulates that were described with reference to tableting, only they are not subjected to a final tableting step.

Yet more particularly, a tablet can, for example, be formulated by blending 100 mg spray dried lactose, 50 mg of losartan potassium treated by the method of the present invention, and 5 mg of magnesium stearate and directly compressing the composition in a tablet machine.

A capsule can, for example, be prepared by filling half of a gelatin capsule with the above tablet composition and capping it with the other half of the gelatin capsule.

Capsules, tablets and lozenges and other unit dosage forms preferably contain a dosage level of about 10 mg to about 100 mg, more preferably from about 25 mg to about 50 mg of losartan potassium treated by the method of the present invention.

The following examples are given for the purpose of illustrating the present invention and shall not be construed as limiting the scope or spirit of the invention.

### EXAMPLES

### Example 1

### Preparation of Losartan Free Acid

Aqueous hydrochloric acid (3 N, 39.1 mL, 117.3 mmol (3 eq.)) was added to a suspension of trityl losartan (26.0 g, 39.1 mmol) in acetone (150 mL) at room temperature. The reaction mixture was stirred for about 5 hours. A solution of potassium hydroxide (85%, 11.0 g, 195.5 mmol, 5 eq.) in water (100 mL) was slowly added and acetone was evaporated under reduced pressure. A slightly yellow precipitate was filtered, washed with water (2x20 mL), and dried under reduced pressure (about 10 mm Hg) at about 50° C. Triphenyl methanol (10.1 g, 99% yield) was recovered in 94.6% purity as determined by HPLC.

Ethyl acetate (100 mL) was added to the aqueous filtrate and the two-phase mixture was vigorously stirred and acidified to pH 3.5-3.6 with slow addition of 3 N aqueous hydrochloric acid (about 25 mL). The resulting suspension was stirred for an additional 30 minutes and filtered. The wet cake was washed with ethyl acetate (50 mL) and an acetone/water (50:50, 50 mL) mixture and dried under reduced pressure for about 2 hours at about 50 °C. Losartan free acid (15.0 g, 91.0% yield) was obtained in 97.68% purity as determined by HPLC.

The phases of filtrate were separated and ethyl acetate phase (yellowish organic phase) was concentrated to 40 mL volume. The precipitate was formed and after about 20 hours of stirring at room temperature, the precipitate was collected by filtration and dried under reduce pressure to yield additional losartan free acid (0.9 g, 5.5% yield).

### Example 2

### Preparation of Losartan Potassium

A solution of potassium hydroxide (0.305 g, 4.62 mmol (1 eq.)) and isopropyl alcohol (15 mL) was slowly added to a suspension of losartan free acid (2.0 g, 4.73 mmol) in isopropyl alcohol (25 mL). The reaction mixture was stirred for about 2 hours at room temperature. The mixture was filtered, concentrated to about a 15 mL volume, heated to reflux and stirred for about 12 hours at room temperature. The precipitate was filtered, washed with isopropyl alcohol (5 mL), and dried under reduced pressure for about 2 hours at about 50° C to give losartan potassium (1.85 g, 85% yield) as a white powder. Yield of losartan potassium staring from losartan trityl was about 78%. Purity was determined to be 99.74% by HPLC. Losartan potassium (1.0 g) was triturated with ethyl acetate (10 mL), having a purity of 99.775% by HPLC.

The overall yield of losartan potassium from trityl losartan was 78%.

### Example 3

### Preparation of Losartan Potassium with Improved Flowability

Dry losartan potassium (50 g) is reslurried in heptane (200 mL) at about 25° C for about 4 hours. The suspension is filtered and dried under vacuum at about 50-60° C for about 10 hours. The Hausner ratio is decreased from about 1.50-1.60 to about 1.30-1.40. Yield is about 98%.

### Example 4

### Preparation of Losartan Potassium with Improved Flowability

Dry losartan potassium (50 g) is reslurried in heptane (500 mL) at about 100° C for about 10 hours. The suspension is cooled to about 25° C, filtered and dried under vacuum at about 50-60° C for about 10 hours. The Hausner ratio is decreased from about 1.50-1.60 to about 1.30-1.35. Yield is about 98%.

### Example 5

### Preparation of Losartan Potassium with Improved Flowability

Dry losartan potassium (50 g) is reslurried in cyclohexane (200 mL) at about 80° C for about 4 hours. The suspension is filtered and dried under vacuum at about 50-60° C for about 10 hours. The Hausner ratio is decreased from about 1.50-1.60 to about 1.3-1.35. Yield is about 98%.

### Example 6

### Preparation of Losartan Potassium with Improved Flowability

Dry losartan potassium (50 g) is reslurried in toluene (200 mL) at about 25° C for about 4 hours. The suspension is filtered and dried under vacuum at about 50-60° C for about 10 hours. The Hausner ratio is decreased from about 1.50-1.60 to about 1.3-1.35. Yield is about 98%.

## Claims

1. A method of increasing the flowability of losartan potassium powder initially having a Hausner ratio of about 1.45 or greater comprising the step of reslurrying the losartan potassium powder in a reslurry solvent selected from the group consisting of the hydrocarbons, the alkyl ethers, the alkyl esters, and mixtures of two or more of these.

2. The method of claim 1 wherein the reslurry solvent is selected from the group consisting of: the hexanes, the heptanes, cyclohexane, methylcyclohexane, benzene, toluene, the xylenes, and mixtures of two or more of them.

3. The method of claim 1 wherein the reslurry solvent is selected from ethyl acetate, propyl acetate, butyl acetate, and mixtures of two or more of these.

4. The method of claim 1 wherein the reslurry solvent is an alkyl ether or a mixture of alkyl ethers.

5. The method of claim4 wherein the reslurry solvent is diethyl ether or dibutyl ether.

6. The method of claim 1 wherein the losartan potassium is prepared by neutralizing losartan free acid with a potassium base in the presence of a protic solvent.

7. The method of claim 6 wherein the protic solvent is an alcohol

8. The method of claim 1 further comprising the steps of isolating and drying losartan potassium after the reslurry to obtain a powder.

9. The method of claim 8 further comprising the step of milling the isolated and dried losartan potassium.

10. A method of increasing the flowability of losartan potassium powder initially having a Hausner ratio of about 1.45 or greater, wherein the losartan potassium is made by neutralizing losartan free acid with a potassium base in the presence of an alcohol, comprising the steps of
a) reslurrying the losartan potassium in a reslurry solvent selected from: the hexanes, the heptanes, cyclohexane, methylcyclohexane, benzene, toluene, the xylenes, ethyl acetate, propyl acetate, butyl acetate, diethyl ether, dibutyl ether, and mixtures of two or more of them.
b) isolating the losartan potassium after reslurring, and
c) drying the losartan potassium isolated after reslurry to obtain a powder, wherein the dried losartan potassium powder has a Hausner ratio less than 1.45.

11. The method of claim 6 or claim 10 wherein the potassium base is potassium hydroxide.

12. The method of claim 7 or claim 10 wherein the alcohol is isopropanol.

13. The method of claim 1 or claim 10 wherein the reslurrying is at about the boiling point of the reslurry solvent.

14. The method of claim 10 wherein the reslurry solvent is selected from: the heptanes, cyclohexane, and toluene.

15. The method of claim 8 or claim 10 further comprising the step of milling the dried losartan potassium powder.

16. The method of claim 8 or claim 10 wherein the isolated dried losartan potassium powder has a Hausner ratio less than 1.45.

17. The method of claim 16 wherein the isolated, dried losartan potassium powder has a Hausner ratio ≤ 1.3.

18. A method of increasing the flowability of losartan potassium powder initially having a Hausner ratio of about 1.45 or greater, wherein the losartan potassium is made by neutralizing losartan free acid with potassium hydroxide in the presence of isopropanol, comprising the steps of:
a) reslurrying the losartan potassium in a reslurry solvent selected from the heptanes, cyclohexane, and toluene, wherein the reslurrying is at about the boiling point of the reslurry solvent,
b) isolating the losartan potassium after reslurrying,
c) drying the losartan potassium isolated after reslurry to obtain a powder, and
d) milling the dried losartan potassium powder to obtain losratan potassium powder having Hausner ratio **≤**1.3.

19. A method of preparing a pharmaceutical composition comprising losartan potassium powder having Hausner ratio < 1.45 which method comprises a method according to any one of claims 1 to 18.

## Patentansprüche

1. Verfahren zur Verbesserung der Fließfähigkeit von Losartankaliumpulver, welches anfänglich einen Hausner-Faktor von etwa 1,45 oder größer aufweist, umfassend die Stufe des Wiederaufschlämmens des Losartankaliumpulvers in einem Wiederaufschlämmlösungsmittel, das ausgewählt ist aus der Gruppe, die aus den Kohlenwasserstoffen, den Alkylethern, den Alkylestern und Gemischen von zwei oder mehreren von diesen besteht.

2. Verfahren nach Anspruch 1, wobei das Wiederaufschlämmlösungsmittel ausgewählt ist aus der Gruppe, die aus: den Hexanen, den Heptanen, Cyclohexan, Methylcyclohexan, Benzen, Toluol, den Xylenen und Gemischen von zwei oder mehreren davon besteht.

3. Verfahren nach Anspruch 1, wobei das Wiederaufschlämmlösungsmittel ausgewählt ist unter Ethylacetat, Propylacetat, Butylacetat und Gemischen von zwei oder mehreren von diesen.

4. Verfahren nach Anspruch 1, wobei das Wiederaufschlämmlösungsmittel ein Alkylether oder ein Gemisch von Alkylethern ist.

5. Verfahren nach Anspruch 4, wobei das Wiederaufschlämmlösungsmittel Diethylether oder Dibutylether ist.

6. Verfahren nach Anspruch 1, wobei das Losartankalium hergestellt wird durch Neutralisieren von freier Losartansäure mit einer Kaliumbase in der Gegenwart eines protischen Lösungsmittels.

7. Verfahren nach Anspruch 6, wobei das protische Lösungsmittel ein Alkohol ist.

8. Verfahren nach Anspruch 1, welches nach dem Wiederaufschlämmen weiterhin die Stufen der Isolierung und Trocknung von Losartankalium umfaßt, um ein Pulver zu erhalten.

9. Verfahren nach Anspruch 8, welches weiterhin die Stufe des Mahlens des isolierten und getrockneten Losartankaliums umfaßt.

10. Verfahren zur Verbesserung der Fließfähigkeit von Losartankaliumpulver, welches anfänglich einen Hausner-Faktor von etwa 1,45 oder größer aufweist, wobei das Losartankalium hergestellt wird durch Neutralisieren von freier Losartansäure mit einer Kaliumbase in der Gegenwart eines Alkohols, umfassend die Stufen, bei denen man:
a) das Losartankalium in einem Wiederaufschlämmlösungsmittel, weiches ausgewählt ist unter: den Hexanen, den Heptanen, Cyclohexan, Methylcyclohexan, Benzen, Toluol, den Xylenen, Ethylacetat, Propylacetat, Butylacetat, Diethylether, Dibutylether und Gemischen von zwei oder mehreren davon, wiederaufschlämmt
b) das Losartankalium nach dem Wiederaufschlämmen isoliert und
c) das nach dem Wiederaufschlämmen isolierte Losartankalium trocknet, um ein Pulver zu erhalten, wobei das getrocknete Losartankaliumpulver einen Hausner-Faktor von weniger als 1,45 aufweist.

11. Verfahren nach Anspruch 6 oder Anspruch 10, wobei die Kaliumbase Kaliumhydroxid ist.

12. Verfahren nach Anspruch 7 oder Anspruch 10, wobei der Alkohol Isopropanol ist.

13. Verfahren nach Anspruch 1 oder Anspruch 10, wobei das Wiederaufschlämmen bei etwa dem Siedepunkt des Wiederaufschlämmlösungsmiftels erfolgt.

14. Verfahren nach Anspruch 10, wobei das Wiederaufschlämmlösungsmittel ausgewählt ist unter: den Heptanen, Cyclohexan und Toluol.

15. Verfahren nach Anspruch 8 oder Anspruch 10, welches weiterhin die Stufe des Mahlens des getrockneten Losartankaliumpulvers umfaßt.

16. Verfahren nach Anspruch 8 oder Anspruch 10, wobei das isolierte, getrocknete Losartankaliumpulver einen Hausner-Faktor von weniger als 1,45 aufweist.

17. Verfahren nach Anspruch 16, wobei das isolierte, getrocknete Losartankaliumpulver einen Hausner-Faktor von ≤ 1,3 aufweist.

18. Verfahren zur Verbesserung der Fließfähigkeit von Losartankaliumpulver, welches anfänglich einen Hausner-Faktor von etwa 1,45 oder größer aufweist, wobei das Losartankalium hergestellt wird durch Neutralisieren von freier Losartansäure mit Kaliumhydroxid in der Gegenwart von Isopropanol, umfassend die Stufen, bei denen man:
a) das Losartankalium in einem Wiederaufschlämmlösungsmittel, welches ausgewählt ist unter den Heptanen, Cyclohexan und Toluol, wiederaufschlämmt, wobei das Wiederaufschlämmen etwa bei dem Siedepunkt des Wiederaufschlämmlösungsmittels erfolgt,
b) das Losartankalium nach dem Wiederaufschlämmen isoliert,
c) das nach dem Wiederaufschlämmen isolierte Losartankalium trocknet, um ein Pulver zu erhalten, und
d) das getrocknete Losartankaliumpulver mahlt, um ein Losartankaliumpulver mit einem Hausner-Faktor von ≤1,3 zu erhalten.

19. Verfahren zur Herstellung einer pharmazeutischen Zusammensetzung umfassend Losartankaliumpulver mit einem Hausner-Faktor ≤ 1,45, wobei das Verfahren ein Verfahren nach einem der Ansprüche 1 bis 18 umfaßt.

## Revendications

1. Une méthode d'amélioration de la fluidité d'une poudre de losartan potassium présentant initialement un rapport Hausner d'environ 1,45 ou supérieur, comprenant l'étape de remise en suspension de la poudre de losartan potassium dans un solvant de remise en suspension choisi dans le groupe consistant en les hydrocarbures, les éthers d'alkyle, les esters d'alkyle et des mélanges de deux ou plus de ceux-ci.

2. La méthode selon la revendication 1, dans laquelle le solvant de remise en suspension est choisi dans le groupe consistant en : les hexanes, les heptanes, le cyclohexane, le méthylcyclohexane, le benzène, le toluène, les xylènes et des mélanges de deux ou plus de ceux-ci.

3. La méthode selon la revendication 1, dans laquelle le solvant de remise en suspension est choisi parmi l'acétate d'éthyle, l'acétate de propyle, l'acétate de butyle et des mélanges de deux ou plus de ceux-ci.

4. La méthode selon la revendication 1, dans laquelle le solvant de remise en suspension est un éther d'alkyle ou un mélange d'éthers d'alkyle.

5. La méthode selon la revendication 4, dans laquelle le solvant de remise en suspension est l'éther diéthylique ou l'éther dibutylique.

6. La méthode selon la revendication 1, dans laquelle le losartan potassium est préparé par la neutralisation de l'acide libre de losartan à l'aide d'une base potassique en présence d'un solvant protique.

7. La méthode selon la revendication 6, dans laquelle le solvant protique est un alcool.

8. La méthode selon la revendication 1, comprenant en outre les étapes d'isolement et de séchage du losartan potassique après la remise en suspension pour obtenir une poudre.

9. La méthode selon la revendication 8, comprenant en outre l'étape de broyage du losartan potassium isolé et séché.

10. Une méthode d'amélioration de la fluidité d'une poudre de losartan potassium présentant initialement un rapport Hausner de 1,45 ou supérieur, dans laquelle on prépare le losartan potassium par neutralisation de l'acide libre de losartan à l'aide d'une base potassique en présence d'un alcool,comprenant les étapes de :
a) remise en suspension du losartan potassium dans un solvant de remise en suspension choisi parmi : les hexanes, les heptanes, le cyclohexane, le méthylcyclohexane, le benzène, le toluène, les xylènes, l'acétate d'éthyle, l'acétate de propyle, l'acétate de butyle, l'éther diéthylique, l'éther dibutylique et des mélanges de deux ou plus de ceux-ci.
b) isolement du losartan potassium après la remise en suspension, et
c) séchage du losartan potassium isolé après remise en suspension pour obtenir une poudre, la poudre de losartan potassium séché en poudre ayant un rapport de Hausner inférieur à 1,45.

11. La méthode selon la revendication 6 ou la revendication 10, dans laquelle la base potassique est l'hydroxyde de potassium.

12. La méthode selon la revendication 7 ou la revendication 10, dans laquelle l'alcool est l'isopropanol.

13. La méthode selon la revendication 1 ou la revendication 10, dans laquelle la remise en suspension s'effectue au voisinage du point d'ébullition du solvant de remise en suspension.

14. La méthode selon la revendication 10, dans laquelle le solvant de remise en suspension est choisi parmi : les heptanes, le cyclohexane, et le toluène.

15. La méthode selon la revendication 8 ou la revendication 10, comprenant en outre l'étape de broyage de la poudre de losartan potassium séchée.

16. La méthode selon la revendication 8 ou la revendication 10, dans laquelle la poudre de losartan potassium isolée séchée présente un rapport de Hausner inférieur à 1,45.

17. La méthode selon la revendication 16, dans laquelle la poudre de losartan potassium isolée séchée présente un rapport de Hausner ≤ 1,3.

18. Une méthode d'amélioration de la fluidité d'une poudre de losartan potassium ayant initialement un rapport de Hausner d'environ 1,45 ou supérieur, dans laquelle on obtient le losartan potassium par neutralisation de l'acide libre à l'aide d'hydroxyde de potassium en présence d'isopropanol, comprenant les étapes de :
a) remise en suspension de losartan potassium dans un solvant de remise en suspension choisi parmi les heptanes, le cyclohexane et le toluène, la remise en suspension s'effectuant au voisinage du point d'ébullition du solvant de remise en suspension,
b) isolement du losartan potassium après remise en suspension,
c) séchage du losartan potassium isolé après remise en suspension pour obtenir une poudre ; et
d) broyage de la poudre de losartan potassium séchée pour obtenir une poudre de losartan potassium présentant un rapport de Hausner ≤ 1,3.

19. Une méthode de préparation d'une composition pharmaceutique comprenant de la poudre de losartan potassium ayant un rapport de Hausner < 1,45, ladite méthode comprenant une méthode selon l'une quelconque des revendications 1 à 18.
